# EUROPEAN PATENT SPECIFICATION

(11) **EP 0 676 413 B1**
(45) Date of publication and mention of the grant of the patent: **05.01.2005**
(21) Application number: 95101560.1
(22) Date of filing: 06.02.1995
(51) Int. Cl.: C07K 14/715, C12N 15/12, C12N 15/63, C07K 16/28, A61K 38/17, G01N 33/53

(54) **Interferon-alpha/beta binding protein, its preparation and use**
Interferon alpha/beta bindendes Protein, seine Herstellung und Anwendung
Protéine liant l'interféron alpha/bêta, sa production et son application

(30) Priority: 07.02.1994 IL 10858494
(43) Date of publication of application: 11.10.1995
(73) Proprietor: YEDA RESEARCH & DEVELOPMENT COMPANY, LTD., 76100 Rehovot (IL)
(72) Inventor: Cohen, Batya, Tel-Aviv (IL); Novick, Daniela, Rehovot (IL); Rubinstein, Menachem, Givat Shmuel (IL)
(74) Representative: VOSSIUS & PARTNER

(56) References cited:
- EP-A- 0 588 177
- WO-A-91/05862
- WO-A-92/18626
- FEBS LETTERS, vol. 314, no. 3, December 1992 pages 445-448, NOVICK, D. ET AL. 'Soluble interferon-alpha receptor molecules are present in body fluids'
- CELL, vol. 77, 6 May 1994 pages 391-400, NOVICK, D. ET AL. 'The human Interferon alpha/beta receptor: ...'

## Description

The present invention relates to novel interferon-α/β binding proteins, capable of modulating the activity of various IFN-α subtypes, as well as the activity of IFN-β. More particularly, this invention relates to the cloning of DNA molecules coding for these proteins and their expression in host cells.

Israel Patent Application No. 103052 describes and claims a soluble IFN-α receptor protein of molecular weight of about 45,000, identified by Western blotting with monoclonal anti-IFN-α receptor antibodies. The above application also describes and claims a different soluble IFN-α binding protein, having a molecular weight of about 40,000, that was identified by crosslinking with ¹²⁵I-IFN-α2 and immuno-precipitation with anti-IFN-α monoclonal antibodies. When obtained from serum, this species had a molecular weight of 50K. Israel Patent Application No. 106591 describes and claims the aforesaid 40,000 IFN-α binding protein, (hereinafter "IFNAB-BP" or "IFNAB-BPII") obtained from urine in a homogenous state and having a sequence that differs from any other known protein. The two above-mentioned Israel Patent Applications correspond to EP-A 0 588 177.

The IFNAB-BP binds to and blocks the activity of a variety of IFN-α subtypes, as well as IFN-β. In this respect the binding characteristics of IFNAB-BP are significantly different from those of a previously described cell surface interferon receptor that responds only to human interferon alpha B.

In accordance with the present invention, two cDNA molecules coding for precursors of IFNAB-BP are cloned and their sequence is determined. Both are probably derived from the same gene, e.g., by alternative splicing. Production of two recombinant proteins, designated IFNAB-BPI and IFNAB-BPII in mammalian and other host cells is also described.

IFNAB-BPI and IFNAB-BPII are capable of modulating the activity of type I interferons, i.e. the various subtypes of interferon-α, as well as interferon-β. Thus they may inhibit undesired effects of type I interferons.

Type I interferons (IFNs) (IFN-α, IFN-β and IFN-ω) constitute a family of structurally related cytokines, usually defined by their ability to confer resistance to viral infections. Many other biological activities of type I IFNs have been reported, including inhibition of cell proliferation, induction of class I MHC antigens and several other immuno-regulatory activities (1). IFN-α and IFN-β are useful for the treatment of several viral diseases, including hepatitis-C (2,3) and viral warts (4,5), as well as certain malignancies such as hairy cell leukemia (6), chronic myelogenous leukemia (7) and Kaposi's sarcoma (8).

IFN-α was detected in sera of various patients having autoimmune diseases such as systemic lupus erythematosus (9), as well as AIDS patients (10). IFN-α was implicated in the progression of juvenile diabetes (11). There has also been a report that increased expression of IFN-∝ in the white matter microglia may contribute to Alzheimer's disease pathology (51). Further, IFN-α therapy has been shown in some cases to lead to undesired side effects, including fever and neurological disorders (12). Hence there are pathological situations in which neutralization of IFN-α activity may be beneficial to the patient.

As in the case of other cytokines, IFN-α exerts its biological activities by binding to a cell surface receptor, which is specific for all IFN-α subtypes, as well as for IFN-β (13). A human IFN-α receptor (IFNAR) was identified and cloned from Daudi cells (14). The cloned receptor has a single transmembrane domain, an extracellular and an intracellular domain. When expressed in murine cells, this receptor confers responsiveness to human IFN-αB but not significantly to other IFN-α and IFN-β species, indicating that additional components may be involved in the response to IFN-β and to various IFN-α subtypes.

Several other studies indicate that there are additional components or receptor subunits involved in the binding of IFN-α and IFN-β (15-17). Furthermore, it was reported that the already described receptor (14) is involved in binding of all IFN-α and IFN-β species (18).

Cytokine binding proteins (soluble cytokine receptors) correspond to the extracellular ligand binding domains of their respective cell surface cytokine receptors. They are derived either by alternative splicing of a pre-mRNA common to the cell surface receptor, or by proteolytic cleavage of the cell surface receptor. Such soluble receptors have been described in the past, including among others, the soluble receptors of IL-6 and IFN-γ (19-21), TNF (22-24), IL-1 (25-27), IL-4 (25,28), IL-2 (29,30), IL-7 (31) and IFN-alpha (32).

The present invention provides DNA molecules encoding the known IFN-α/β binding protein (IL106591). Such DNA molecules actually encode two distinct proteins, IFNAB-BPI and IFNAB-BPII, probably derived from the same pre-mRNA by alternative splicing, to yield two mRNA molecules, one having a size of about 1.5 kb and the other a size of about 4.5 kb, each of which encodes one of the binding proteins, the 1.5kb mRNA encoding the IFNAB-BPI and the 4.5 kb mRNA encoding the IFNAB-BPII. The term IFNAB-BP corresponds to both IFNAB-BPI and IFNAB-BPII. Urinary IFNAB-BP is identified as IFNAB-BPII.

Accordingly, the present invention provides a DNA and RNA molecule encoding an IFN-α/β binding protein selected from IFNAB-BPI, IFNAB-BPII, precursors and fused proteins of IFNAB-BPI and IFNAB-BPII.

The invention further provides replicable expression vehicles containing said DNA molecules, hosts transformed therewith and proteins produced by such transformed hosts. The term "DNA molecules" includes genomic DNA, cDNA, synthetic DNA and combinations thereof. encode proteins having the same biological activity as the IFNAB-BPs.

The present invention also provides methods for preparation in host cells capable of production of a functional IFNAB-BPI and IFNAB-BPII, precursors or fused proteins.

The present invention also provides the recombinant IFNAB-BPI and IFNAB-BPII, precursors or, fused proteins, and salts of all of same, and pharmaceutical compositions containing IFNAB-BPI or IFNAB-BPII, precursors fused proteins, or salts of all of same.

The invention further provides a diagnostic composition comprising
(a) the DNA molecule of the invention; and/or
(b) the IFN-α/β binding protein of the invention.

Said diagnostic compositions may be used in the detection of the protein of the invention.

IFNAB-BPI and IFNAB-BPII inhibit the biological activities of natural human leukocyte and fibroblast interferons, as well as recombinant human IFN-α2, IFN-αB, IFN-αC and IFN-β. IFNAB-BPI corresponds to a novel transmembrane protein which is the ligand-binding IFN-α/β receptor. IFNAB-BPII is a soluble receptor, essentially corresponding to the extracellular, ligand-binding domain of IFNAB-BPI.

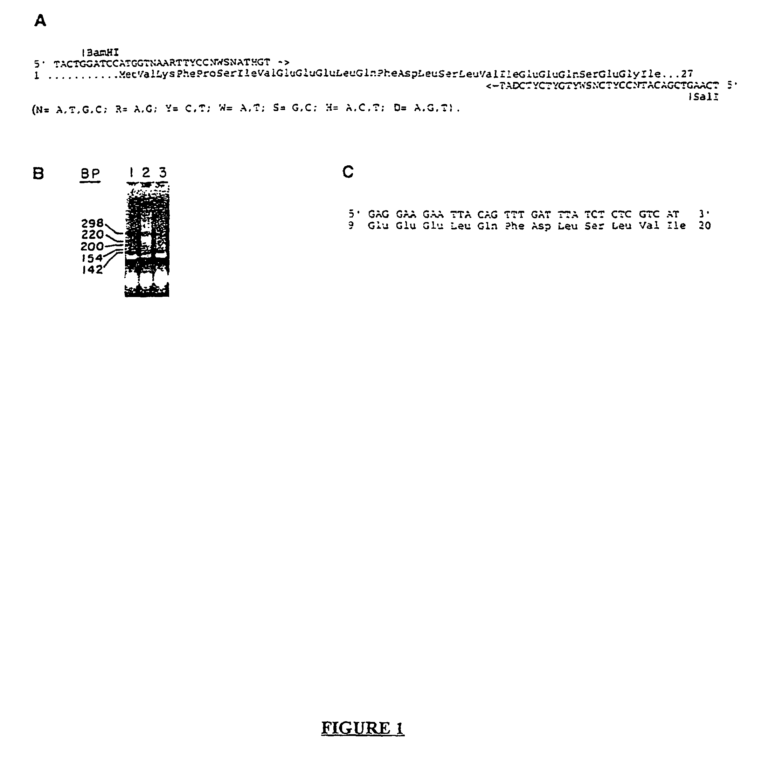

FIGURE 1 shows the cloning strategy of IFNAB-BPI and IFNAB-BPII:
   (A) Middle row: The sequence of an internal CNBr peptide (27 amino acid residues, cb7) obtained from the urinary 40,000 IFNAB-BP.
      Top and bottom rows: Synthetic sense (top) and antisense (bottom), degenerate oligonucleotide mixtures, made on the basis of the peptide sequence and used for reverse transcription (antisense primer only) and for polymerase chain reaction (PCR).
   (B) Agarose gel electrophoresis of PCR products, made with the above sense and antisense primers. The following RNAs and primers were used for generating cDNA that was used as template for the PCR: (1) Poly A + RNA of Daudi cells, antisense primer. (2) Poly A + RNA of Daudi cells, oligo d(T) primer. (3) Total RNA of WISH cells, antisense primer. The size (bp) of the DNA markers is indicated on the left side.
   (C) Top row: The non-degenerate portion of the sequence, obtained from pBluescript clones of the 101 bp PCR product.
      Bottom row: Translation of the resulting non-degenerate DNA sequence into the expected sequence which is part of the sequence of peptide cb7 (residues 9-20).

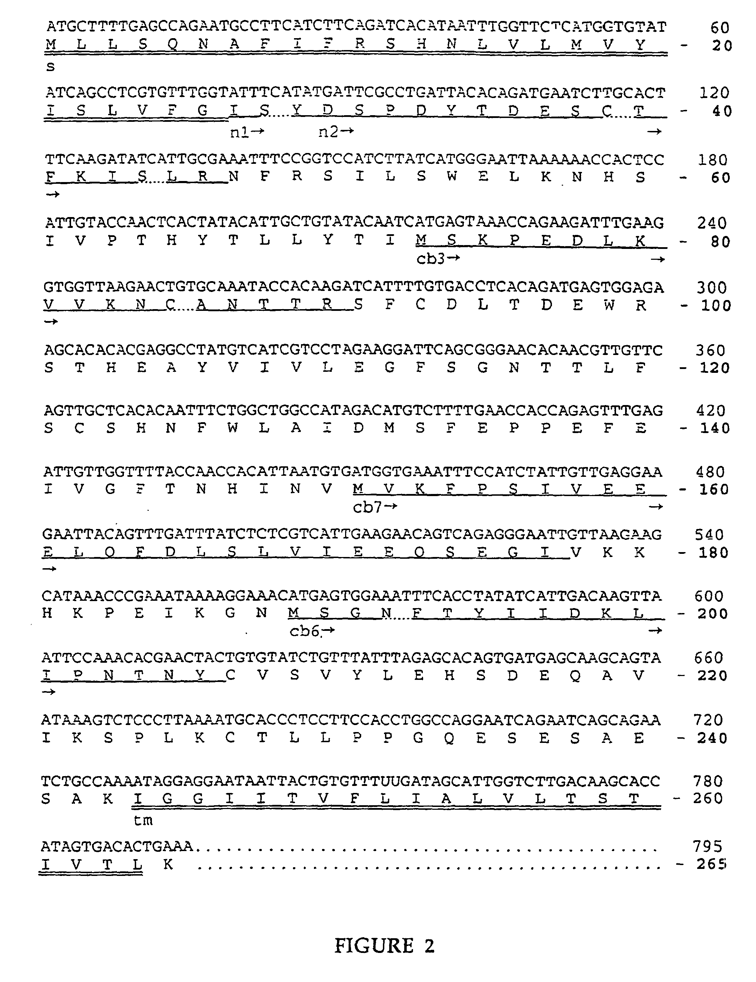

FIGURE 2 shows the cDNA and translated polypeptide sequence of clone q10, carrying the cDNA of IFNAB-BPI:
   This clone was isolated from a lambda gt11 library, made from cDNA of human HeLa cells, by screening with a synthetic oligonucleotide corresponding to the non-degenerate DNA sequence of Figure 1(C). Sequences corresponding to the N-terminus of the urinary IFNAB-BP and to its CNBr peptides are underlined and the corresponding sequence name is given below the line (nl, N-terminus 1; n2, N-terminus 2; cb3, CNBr peptide 3; cb6, CNBr peptide 6; cb7, CNBr peptide 7). Hydrophobic sequences, corresponding to the signal peptide(s) and the transmembrane domain (tm) are double underlined. Bold numbers on the right side are those of the amino acid residues. Plain numbers correspond to nucleotide residues, taking the initiator A of ATG as No. 1.

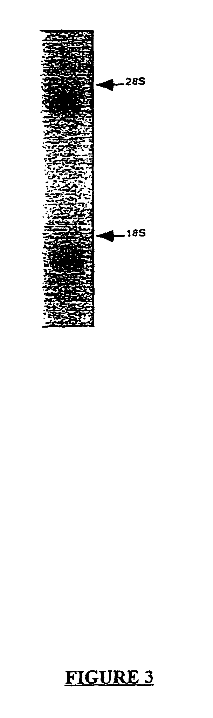

FIGURE 3 shows detection of mRNA by Northern blotting with a specific probe, common to the sequence of IFNAB-BPI and IFNAB-BPII:
   A 397 base pair (bp) probe, corresponding to nucleotides 218-614 of IFNAB-BPI was prepared by polymerase chain reaction with appropriate primers and [³²P] labeled by random primer labeling. Poly A+ RNA from human Daudi cells was analyzed by electrophoresis on agarose (1.5%), blotted onto nitrocellulose and hybridized with the specific probe. The size of the ribosomal RNA is indicated on the right side.

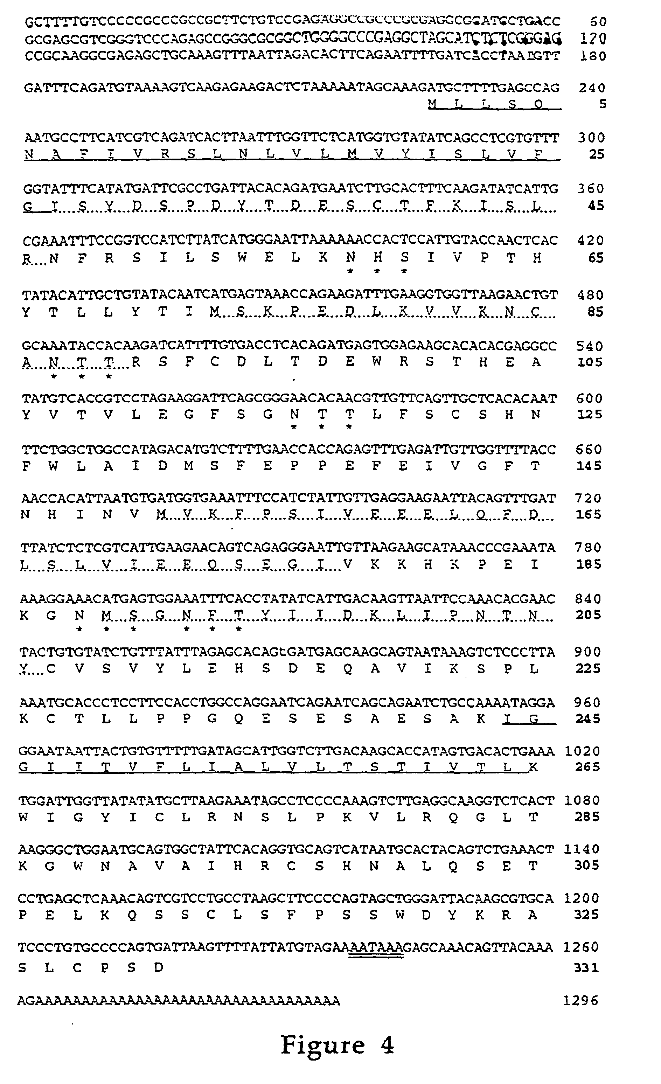

FIGURE 4 shows nucleotide and amino acid Sequences of a complete 1.5kb cDNA clone corresponding to IFNAB-BPI. Amino acid residues in single letter codes are numbered in bold, starting at the translation-initiation codon. Hydrophobic leader and transmembrane regions are underlined. N-terminal protein sequences of urinary IFNAB-BP (from codon 27) and the internal CNBr peptides are dot-underlined (however Cys and N-glycosylated Asn residues are not detectable). N-glycosylation signals are indicated by asterisks and the polyadenylation signal is double underlined.

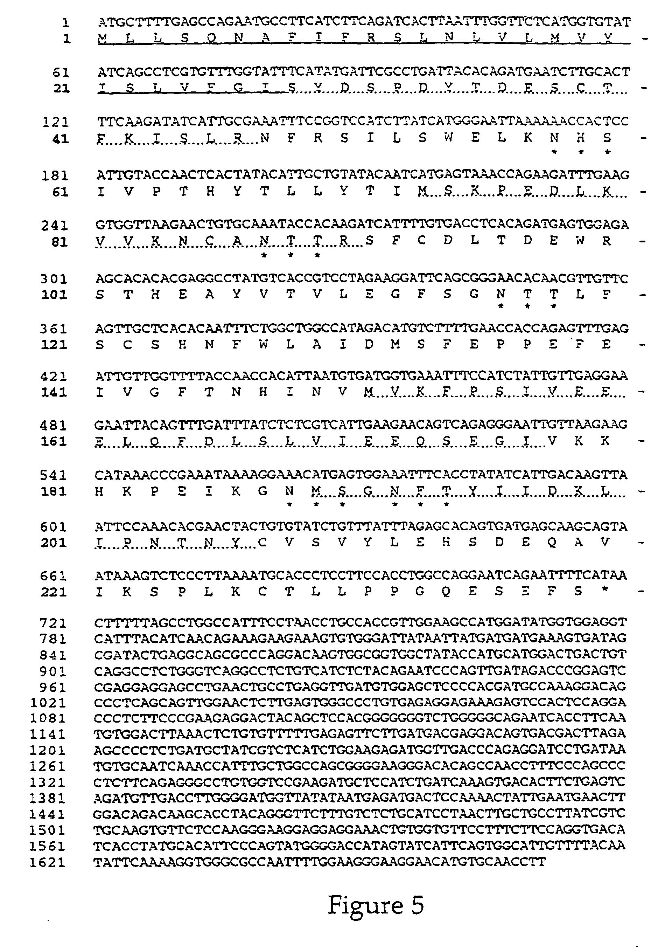

FIGURE 5 shows partial nucleotide and amino acid Sequences of a 4.5kb cDNA clone corresponding to IFNAB-BPII. Amino acid residues in single letter codes are numbered in bold, starting at the translation-initiation codon. The hydrophobic leader region is underlined. N-terminal protein sequences of urinary IFNAB-BP (from codon 27) and the internal CNBr peptides are dot-underlined (Cys and N-glycosylated Asn residues are not detectable). N-glycosylation signals and the stop codon are indicated by asterisks.

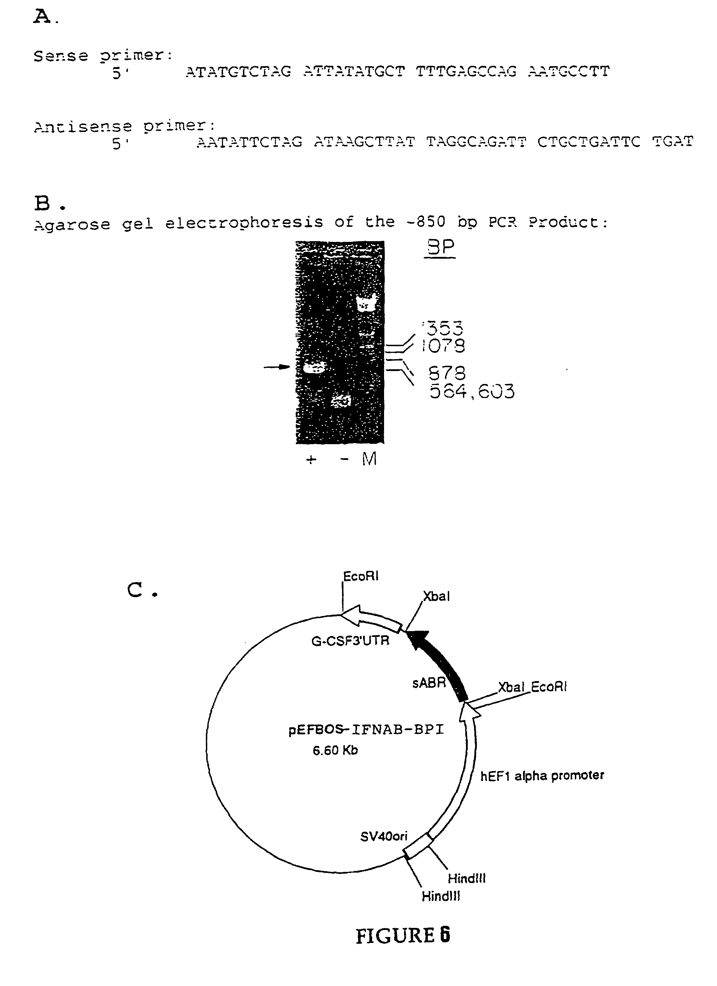

FIGURE 6 shows the construction of a mammalian expression vector for expression of the extracellular, ligand-binding domain of IFNAB-BPI.
   (A) Sense and antisense synthetic oligonucleotides used for preparing the DNA that codes for the extracellular, ligand-binding domain of IFNAB-BPI by polymerase chain reaction.
   (B) Agarose gel electrophoresis of the ~850 bp product of a polymerase chain reaction (PCR), made with the above sense and antisense primers and DNA of clone q10.
   (C) The structure of pEF-BOS-IFNAB-BPI, a mammalian expression vector for production of a soluble IFNAB-BPI.

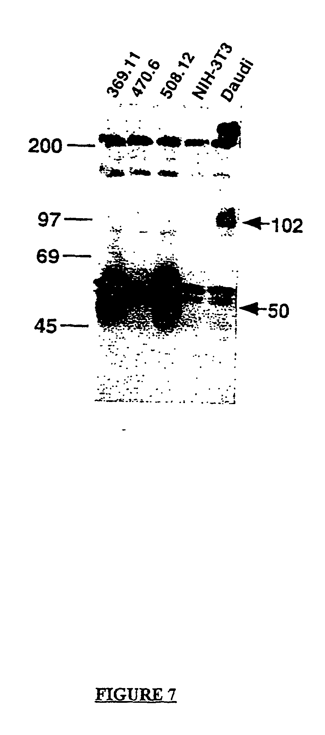

FIGURE 7 shows the expression of IFNAB-BPI and IFNAR in various cells:
   Expression of IFNAB-BPI in various cells is shown by SDS-PAGE (7.5% acrylamide, non-reducing conditions) of detergent cell extracts, followed by immunoblotting with rabbit anti IFNAB-BPII antibody and ¹²⁵I-protein A. Clone 369.11 is NIH-3T3 cells, expressing IFNAB-BPI; Clone 470.6 is NIH-3T3 cells expressing IFNAR; and Clone 508.12 expresses both proteins. Control NIH-3T3 cells and human Daudi cells are shown as well. The 51 kDa form (in murine cells) and 102 kDa form (in Daudi cells) of IFNAB-BPI are indicated by arrows. Molecular mass markers are shown on the left side.

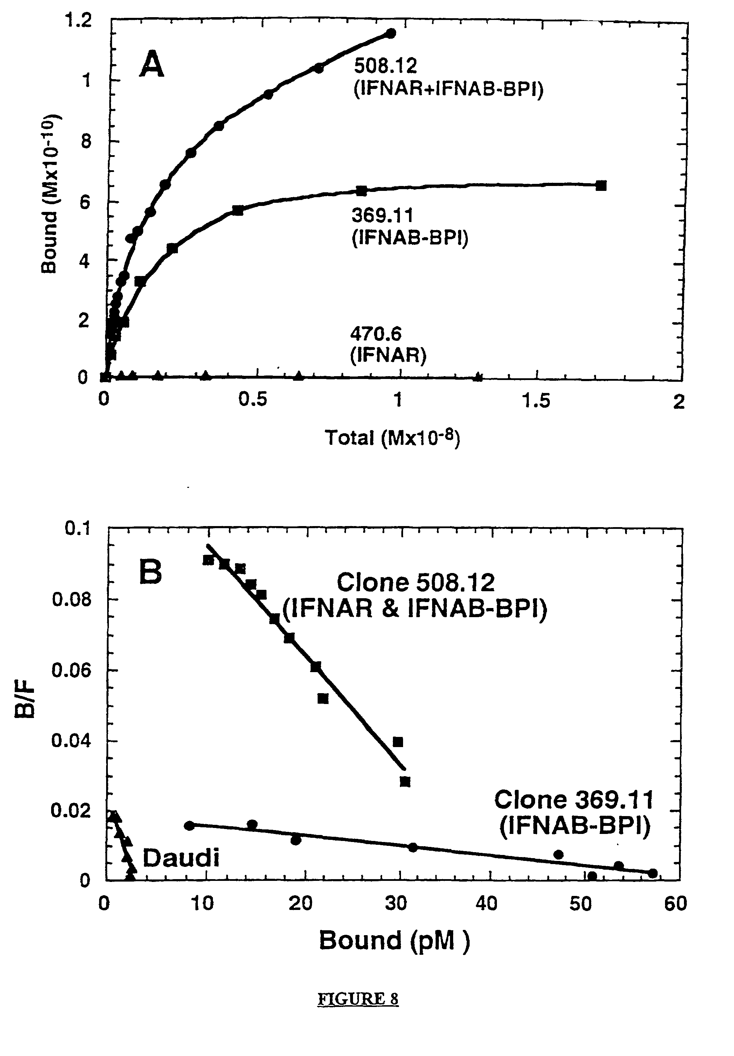

FIGURE 8 shows binding of ¹²⁵I-IFN-α2 to various host cells:
   (A) Saturation binding of ¹²⁵I-IFN-α2 to NIH-3T3 cells expressing IFNAB-BPI (Clone 369.11, ■) and cells expressing both IFNAB-BPI and IFNAR (Clone 508.12, •) and lack of binding to cells expressing IFNAR only (Clone 470.6, Δ). (B) Scatchard analysis of ¹²⁵I-IFN-α2-binding to the above cells. Binding data were analyzed by the LIGAND program. The following cells showed high affinity saturable binding: huDaudi (Δ), IFNAB-BPI-positive cells (Clone 369.11, •) and Clone 508.12, expressing both IFNAR and IFNAB-BPI (■).
FIGURE 9 summarizes the results of a study on a BIAcore system which determine the affinity of urinary IFNAB-BPII to IFN-α2:
   IFN-α2 was immobilized on the sensor chip and various concentrations of urinary IFNAB-BPII were passed through the sensor chip. "Relative response vs. time" shows the binding and dissociation process. The apparent dissociation constant is 3.12x10⁻⁹ M.
FIGURE 10 shows an ELISA of urinary IFNAB-BPII:
   Pure urinary IFNAB-BPII was diluted twofold serially to the indicated concentrations, added to microELISA plates that had been pre-coated with mab anti IFNAB-BPII antibody. The plates were then reacted with rabbit anti IFNAB-BPII antibody followed by a goat anti-rabbit horseradish peroxidase conjugate and ABTS/H₂O₂ substrate. The plates were read at 405/630 nm. The lower limit of detection is 30 pg/ml.

According to Israeli Patent Application No. 106591, an IFN-α/β binding protein having a molecular weight of 40,000 (IFNAB-BP) was isolated from normal urine by two chromatographic steps. Crude urinary proteins were loaded on a column consisting of IFN-α2 bound to agarose. The column was washed to remove non-relevant proteins and the bound proteins were then eluted at low pH. Eluted proteins were then resolved by size exclusion HPLC to give several protein peaks, one of which was characterized by its ability to react specifically with ¹²⁵I-IFN-α2 and to block the antiviral activity of IFN-α and IFN-β. This protein was further characterized by N-terminal microsequence analysis, which gave a major sequence at its N-terminal domain:

Asp-Ser-Pro-Asp-Tyr-Thr-Asp-Glu-Ser-Arg-Thr-Phe-Lys-Ile-Arg-
Leu-Arg.

A minor polypeptide sequence, corresponding to the major sequence, but having three extra amino acid residues (Ile-XXX-Tyr) at the N-terminus of the above sequence, was detected (XXX denotes an unidentified amino acid). The resulting sequence was compared with and found to be completely different from that of the known IFN-αB receptor (14). It was also different from any other known protein and it was not coded by any known DNA sequence, as determined by comparing it to Swissprot and Genebank data libraries, using the FastA program (33).

A sample of the urinary IFNAB-BP was digested with CNBr, resolved on SDS-PAGE, electroblotted onto a PVDF membrane and the resulting digestion fragments were subjected to protein microsequencing. One of the fragments had a molecular weight of less than 10K and an internal sequence as follows (Met precedes the actual sequence):

Met-Val-Lys-Phe-Pro-Ser-Ile-Val-Glu-Glu-Glu-Leu-Gln-Phe-Asp-
Leu-Ser-Leu-Val-Ile-Glu-Glu-Gln-Ser-Glu-Gly-Ile (27 residues).

This internal sequence was reverse-translated into sense and antisense primers to which suitable restriction sites were added. Total RNA was purified from human cells and first strand cDNA was generated with reverse transcriptase, using either the antisense oligonucleotide mixture or oligo d(T) as a primer. The resulting cDNA fragment was then amplified in a polymerase chain reaction (PCR), using the combined sense and antisense degenerate primers. Analysis of the PCR products on a 3% agarose gel showed a specific 101 bp oligonucleotide band. This DNA was restriction-digested, cloned into pBluescript (Stratagene) and competent E.coli were transfected with this vector. Several independent clones were sequenced. The sequence of the region flanked by the sense and antisense degenerate primers was invariant and encoded the expected sequence from the above mentioned CNBr peptide (cb7) of urinary IFNAB-BP. An oligonucleotide corresponding to this non-degenerate internal sequence was synthesized, end-labeled and used for screening of cDNA libraries.

Screening of a lambda gt11 cDNA library of human HeLa cells (Clontech) gave several positive clones. One of these clones designated q10, had an open reading frame that corresponded to a signal peptide, an extracellular domain, a transmembrane domain and a short cytoplasmic domain. The peptide sequences obtained from the urinary IFNAB-BP were all present within the extracellular domain encoded by q10. A few amino acid residues of the peptide sequence were incorrect due to limitation of the protein sequencing technology (mainly the inability to identify Cys and the low levels of peaks corresponding to Ser).

Sense and antisense primers, corresponding to ends of the nucleotide sequence 219-613 of clone q10 (Figure 2) were used for preparing a specific probe by PCR, using clone q10 as a DNA template. The resulting DNA was labeled with [³²P] and used for Northern blot hybridization of poly A+ mRNA from two human cell lines. In both cases two specific bands were observed, one corresponding to 1.5 kb mRNA and another one, corresponding to 4.5 kb mRNA. The primary translation product of the 1.5 kb mRNA is designated as IFNAB-BPI precursor. The primary translation product of the 4.5 kb mRNA is designated as IFNAB-BPII precursor.

The aforementioned specific probe was used for screening an additional human cDNA library and two groups of cDNA clones were identified. One group (about 20 individual clones) had a length of 1.5 kb and coded for the same precursor of the transmembrane protein that was coded by clone q10. The second group (2 individual clones) had a length of 4.5 kb. These sizes were the same as those of the two mRNA species, and therefore the 1.5 kb cDNA clones coded for IFNAB-BPI while the 4.5 kb cDNA clones coded for IFNAB-BPII. Sequencing of the 4.5 kb clones indicated that they code for a precursor of a truncated soluble receptor corresponding to codons 1-239 of clone q10. However codons 238 and 239 were different and were followed by a stop codon. Protein sequence analysis of the C-terminus of the urinary 40,000 IFNAB-BP indicated that it is coded by the 4.5 kb cDNA, as determined by the last two amino acid residues, and hence the urinary IFNAB-BP was identified as IFNAB-BPII. "Precursor" as used herein, is defined as the primary translation product which includes the signal peptide.

DNA coding for the precursor of a truncated soluble form of IFNAB-BPI was generated by PCR. The resulting PCR product was inserted into a mammalian expression vector and used for transfection of various mammalian cells, such as monkey COS cells. Such cells expressed high levels of biologically active recombinant soluble IFNAB-BPI.

Similarly, DNA coding for the precursor of IFNAB-BPII was generated by PCR. The resulting PCR product was inserted into a mammalian expression vector and used for transfection of various mammalian cells, such as monkey COS cells. Such cells expressed high levels of biologically active recombinant IFNAB-BPII.

Similarly, DNA coding for the entire precursor of IFNAB-BPI was generated by PCR. The resulting PCR product was inserted into a mammalian expression vector and used for transfection of various mammalian cells, such as mouse NIH-3T3 cells. Such cells expressed high levels of human IFNAB-BPI. The cells were able to bind human IFN-α2 with a high affinity (*Kd* = 3.6x10⁻⁹ M). When both human IFNAB-BPI and the previously cloned human IFN-αB receptor IFNAR (14) were co-expressed in mouse NIH-3T3 cells the affinity of the composite receptor was increased by about 10-fold (*Kd* = 4x10⁻¹⁰ M). In contrast, when only human IFNAR was expressed in mouse cells no binding of human IFN-α2 could be demonstrated. Therefore, a composite protein containing two attached polypeptides, one of which having the ligand binding domain of IFNAB-BPI or IFNAB-BPII, and the second polypeptide having the extracellular domain of IFNAR, will exhibit a higher affinity for IFN-α as compared with IFNAB-BPI or IFNAB-BPII alone.

The affinity of the urinary IFNAB-BPII for human IFN-α2 was determined by the BIAcore system (Pharmacia, Sweden). IFN-α2 was immobilized on a sensor chip and allowed to bind IFNAB-BPII. Based on Kon and Koff values, a *Kd* value of 3.12x10⁻⁹ M was obtained. this value is very close to the one obtained with NIH-3T3 cells expressing IFNAB-BPI.

The above-mentioned cloning, clone isolation, identification, characterization and sequencing procedures are described in more detail hereinafter in the Examples.

IFNAB-BPI and IFNAB-BPII can also be produced by other types of recombinant cells such as prokaryotic cells, e.g., E.coli, or other eukaryotic cells, such as CHO, yeast or insect cells. Methods for constructing appropriate vectors, carrying DNA that codes for either IFNAB-BPI or IFNAB-BPII and suitable for transforming (e.g., E. Coli and yeast cells) or infecting insect cells in order to produce recombinant IFNAB-BPI and IFNAB-BPII are well known in the art. See, for example, Ausubel et al., eds. "Current Protocols in Molecular Biology" Current Protocols, 1993; and Sambrook et al., eds. "Molecular Cloning: A Laboratory Manual", 2nd ed., Cold Spring Harbor Press, 1989.

The invention further relates to fused proteins consisting of wild type IFNAB-BPI or IFNAB-BPII, fused to another polypeptide or protein and exhibiting a similar ability to block the biological activities of IFN-α and IFN-β or other cytokines which share the interferon alpha/beta receptor.

DNA encoding IFNAB-BPI or IFNAB-BPII, precursors, or fused proteins, and the operably linked transcriptional and translational regulatory signals, are inserted into eukaryotic vectors which are capable of integrating the desired gene sequences into the host cell chromosome. In order to be able to select the cells which have stably integrated the introduced DNA into their chromosomes, one or more markers which allow for selection of host cells which contain the expression vector is used. The marker may provide for prototrophy to an auxotropic host, biocide resistance, e.g., antibiotics, or resistance to heavy metals, such as copper, or the like. The selectable marker gene can either be directly linked to the DNA gene sequences to be expressed, or introduced into the same cell by cotransfection. Additional elements may also be needed for optimal synthesis of single chain binding protein mRNA. These elements may include splice signals, as well as transcription promoters, enhancers, and termination signals (34).

For the purposes of expression of the IFNAB-BPI and IFNAB-BPII proteins, the DNA molecule to be introduced into the cells of choice will preferably be incorporated into a plasmid or viral vector capable of autonomous replication in the recipient host.

Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in E.Coli, for example, pBR322, ColE1, pSC101, pACYC 184, etc. (35); Bacillus plasmids such as pC194, pC221, pT127, etc. (36); Streptomyces plasmids including pIJ101 (37), Streptomyces bacteriophages such as ΦC31 (38) and Pseudomonas plasmids (39,40).

Preferred eukaryotic plasmids include EPV, vaccinia, SV40, 2-micron circle, etc., or their derivatives. Such plasmids are well known in the art (41-45).

Once the vector or DNA sequence containing the construct(s) has been prepared for expression, the expression vector may be introduced into an appropriate host cell by any of a variety of suitable means, such as transformation, transfection, lipofection, conjugation, protoplast fusion, electroporation, calcium phosphate precipitation, direct microinjection, etc.

Host cells to be used in this invention may be either prokaryotic or eukaryotic. Preferred prokaryotic hosts include bacteria such as E.coli, Bacillus, Streptomyces, Pseudomonas, Salmonella, Serratia, etc. The most preferred prokaryotic host is E.coli. Bacterial hosts of particular interest include E.coli K12 strain 294 (ATCC 31446), E.coli X1776 (ATCC 31537), E.coli W3110 (F⁻, lambda⁻, phototropic (ATCC 27325)), and other enterobacteria such as Salmonella typhimurium or Serratia narcescens and various Pseudomonas species. Under such conditions, the protein will not be glycosylated. The prokaryotic host must be compatible with the replicon and control sequences in the expression plasmid.

However, since IFNAB-BPI and IFNAB-BPII are glycosylated proteins, eukaryotic hosts are preferred over prokaryotic hosts. Preferred eukaryotic hosts are mammalian cells, e.g., human, monkey, mouse and Chinese hamster ovary (CHO) cells, because they provide post-translational modifications to protein molecules including correct folding, correct disulfide bond formation, as well as glycosylation at correct sites. Also yeast cells and insect cells can carry out post-translational peptide modifications including high mannose glycosylation. A number of recombinant DNA strategies exist which utilize strong promoter sequences and high copy number of plasmids, which can be utilized for production of the desired proteins in yeast and in insect cells. Yeast cells recognize leader sequences on cloned mammalian gene products and secrete peptides bearing leader sequences. After the introduction of the vector, the host cells are grown in a selective medium, which selects for the growth of vector-containing cells. Expression of the cloned gene sequence(s) results in the production of IFNAB-BPI or IFNAB-BPII, precursors or fusion proteins. The expressed proteins are then isolated and purified by any conventional procedure involving extraction, precipitation, chromatography, electrophoresis, or the like, or by affinity chromatography, using anti-IFNAB-BPI monoclonal antibodies immobilized on a gel matrix contained within a column. Crude preparations containing said recombinant IFNAB-BPI or IFNAB-BPII, on their precursors are passed through the column whereby IFNAB-BPI or IFNAB-BPII, or their precursors will be bound to the column by the specific antibody while the impurities will pass through. After washing, the protein is eluted from the gel under conditions usually employed for this purpose, i.e. at a high or a low pH, e.g. pH 11 or pH 2.

The term "fused protein" refers to a polypeptide comprising IFNAB-BPI or IFNAB-BPII, fused with another protein which, e.g., has an extended residence time in body fluids. IFNAB-BPI or IFNAB-BPII may thus be fused to another protein, polypeptide or the like, e.g., an immunoglobulin or a fragment thereof.

The term "salts" herein refers to both salts of carboxyl groups and to acid addition salts of amino groups of IFNAB-BPI, IFNAB-BPII, their active fractions, muteins, or fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid. Of course, any such salts must have substantially similar activity to IFNAB-BPI or IFNAB-BPII or their active fractions.

The present invention further relates to pharmaceutical compositions comprising a pharmaceutically acceptable carrier and IFNAB-BPI or. IFNAB-BPII of the invention or their precursors, fused proteins and their salts.

The pharmaceutical compositions of the invention are prepared for administration by mixing IFNAB-BPI or IFNAB-BPII with physiologically acceptable carriers, and/or stabilizers and/or excipients, and prepared in dosage form, e.g., by lyophilization in dosage vials. The method of administration can be via any of the accepted modes of administration for similar agents and will depend on the condition to be treated, e.g., intravenously, intramuscularly, subcutaneously, by local injection or topical application, or continuously by infusion, etc. The amount of active compound to be administered will depend on the route of administration, the disease to be treated and the condition of the patient. Local injection, for instance, will require a lower amount of the protein on a body weight basis than will intravenous infusion.

As mentioned in Israel Patent Application No. 106591, the IFN-α/β binding protein (or herein designated IFNAB-BPII) inhibited the antiviral activity of IFN-α2, IFN-αB, IFN-αC and IFN-β and not IFN-γ, which indicates that IFNAB-BPI and IFNAB-BPII are general type I IFN binding proteins. Thus these are useful for modulating or blocking the biological activities of various IFN-α subtypes and IFN-β, for example in type I diabetes, various autoimmune diseases, graft rejections, AIDS and similar diseases, in which there is an aberrant expression of IFN-α or IFN-β, i.e. IFNAB-BPI and IFNAB-BPII may be used in any condition where an excess of IFN-α or IFN-β is endogenously produced or exogenously administered.

Accordingly, IFNAB-BPI and IFNAB-BPII, their precursors fused proteins and their salts are indicated for the treatment of autoimmune diseases, for other inflammations in mammals, for treatments of toxicity caused by administration of interferon alpha or beta, for juvenile diabetes, for lupus erythematosus and for AIDS.

As indicated above, the proteins of the present invention also have non-therapeutic utility such as in the purification of type I interferon species.

The present invention also provides DNA molecules encoding any of the proteins of the present invention as defined above, replicable expression vehicles comprising any such DNA molecules, host cells transformed with any such expression vehicles including prokaryotic and eukaryotic and host cells, preferably monkey COS cells.

The invention also includes a process for the production of any of the proteins of the present invention by culturing a transformed cell in accordance with the present invention and recovering the protein encoded by the DNA molecule and the expression vehicle within such transformed host cell.

The invention will now be illustrated by the following nonlimiting examples:

### EXAMPLE 1: Protein sequence analysis of urinary IFNAB-BP

Pure IFNAB-BP, obtained as described in Israeli Patent Application No 106591 was adsorbed on a PVDF membrane (Pro-Spin, Applied Biosystems, USA) and the membrane was subjected to protein sequence analysis on a Model 475 microsequencer (Applied Biosystems, USA). The following major sequence was obtained:

Asp-Ser-Pro-Asp-Tyr-Thr-Asp-Glu-Ser-Arg-Thr-Phe-Lys-Ile-Arg-Leu-
Arg
1 . . . 5 . . . . 10 . . . . 15 . .

In addition, a secondary polypeptide having three additional amino acid residues (Ile-xxx-Tyr) at the N-terminus of the major sequence was detected (xxx denotes an unidentified amino acid. The resulting sequence is completely different from that of the already known IFN-αB receptor (IFNAR, reference 14) and is different from any other known protein. It is also different from any protein coded by a known DNA sequence, as determined by searching Swissprot and Genebank databases by the program FastA (33). Hence this protein is a novel IFN-α binding protein. Upon isolation of cDNA clones (see below), it was clarified that residue 10 is Cys and not Arg and residue 15 is Ser and not Arg. Furthermore, xxx was identified as Ser. It is known that Cys cannot be identified by the protein microsequencer, while sometimes Ser is destroyed in the analytical process and therefore it is not identified.

A sample of the urinary IFNAB-BP was digested with CNBr, resolved on SDS-PAGE and blotted onto a PVDF membrane. Seven discrete peptide bands, designated cb1 - cb7, were resolved and detected on the membrane upon staining with Coomassie blue. Each band was excised and subjected to protein microsequencing. One of the peptides, cb7, was smaller than 10,000 and gave the following internal sequence (Met precedes the actual sequence):

Met-Val-Lys-Phe-Pro-Ser-Ile-Val-Glu-Glu-Glu-Leu-Gln-Phe-Asp-
Leu-
 1 . . . 5 . . . . 10 . . . . 15 .
Ser-Leu-Val-Ile-Glu-Glu-Gln-Ser-Glu-Gly-Ile....
 . . . 20 . . . . 25 . 27

Another peptide, cb3, had the following sequence (Met precedes the actual sequence):

Met-Ser-Lys-Pro-Glu-Asp-Leu-Lys-Val-Val-Lys-Asn-XXX-Ala-Asn-
1 . . . 5 . . . . 10 . . . . 15
Thr-Thr-Arg....
. . 18

Residue 13 was later identified as Cys, as determined from the cDNA sequence (see below). Cys residues cannot be identified by protein microsequencing.

Another peptide, cb6, had the following sequence (Met precedes the actual sequence):

Met-Ser-Gly-XXX-Phe-Thr-Tyr-Ile-Ile-Asp-Lys-Leu-Ile-Pro-Asn-
 1  .   .   .   5   .   .   .   .  10   .   .   .   .  15
Thr-Asn-Tyr....
 .   .  18

Residue 4 was later identified as Asn, as determined from the cDNA sequence (see below). This Asn residue is part of a potential glycosylation signal sequence (Asn-Phe-Thr) and the absence of Asn signal in the protein sequence indicates that it is indeed glycosylated.

The other peptide bands were identified by sequencing as products of incomplete digestion with CNBr. They gave either the N-terminal domain sequence as previously found for IFNAB-BP or the same internal sequences of cb3, cb6 or cb7.

### EXAMPLE 2: Protein sequence analysis of the C-terminal peptide of urinary IFNAB-BP

A sample of urinary IFNAB-BP (~10 µg) was reduced by DTT, alkylated by iodoacetamide and digested with endoproteinase Lys C (Boehringer Mannheim, Germany) at a 1:50 enzyme to substrate ratio. The resulting peptide mixture was resolved by RP-HPLC, on an RP18 column (Aquapore RP18, Applied Biosystems Inc.) using a gradient of acetonitrile in aq. 0.1% trifluoroacetic acid. Individual peptide peaks were covalently attached to Sequalon AA membranes (Millipore , Bedford MA) and subjected to N-terminal sequencing as above. One of the peptides was identified as the C-terminal peptide having the following sequence:

Cys.Thr.Leu.Leu.Pro.Pro.Gly.Gln.Glu.Ser.Glu.Phe.Ser
1...............5.....................10..........13

The C-terminal sequence corresponded to that of the 4.5 kb cDNA clone (see below) and could be distinguished from that of the putative protein coded by the 1.5 kb cDNA by the last two amino acid residues (Phe12-Ser13 instead of Ser-Ala). Hence the soluble receptor isolated from urine is identified as IFNAB-BPII. It is translated independently from a specific 4.5 kb mRNA and is not formed by shedding of the cell-surface receptor.

### EXAMPLE 3: Construction of degenerate sense and antisense primers and identification of a non-degenerate sequence of IFNAB-BP CDNA.

The sequence of peptide cb7 was reverse-translated into sense (amino acids 1-8) and antisense (amino acids 27-20) primers. Decanucleotides and nonanucleotides, containing the BamH I and Sal I endonuclease restriction sequences, respectively, were added to the 5' ends of the primer oligonucleotides (Figure 1A). Total RNA was extracted from Daudi and WISH cells and first strand cDNA was generated with reverse transcriptase, using either the antisense oligonucleotide mixture or oligo d(T) as a primer. The resulting cDNA fragment was then amplified in a polymerase chain reaction (PCR), using the combined sense and antisense degenerate primers. Analysis of the PCR products on a 3% agarose gel showed the expected 101 bp band, obtained with the cDNA of both Daudi and WISH cells (Figure 1B). The 101 bp fragment was restricted with BamH I and Sal I, cloned into pBluescript II KS+ (Stratagene) and 5 clones were sequenced. The sequence of the region flanked by the sense and antisense primers was invariant and encoded the expected sequence of amino acid residues 9-19 of peptide cb7 (Figure 1C). A 35 bp oligonucleotide, corresponding to the non-degenerate internal sequence was then synthesized and used for screening of cDNA libraries.

### Example 4. Identification of partial cDNA clones of IFNAB-BPI

The synthetic 35 bp non-degenerate oligonucleotide of Example 2 was [³²P] labeled and used for screening of a lambda gt11 cDNA library of human HeLa cells (Clontech). Five positive clones were identified. One of these clones, named q10, contained an insert of 1.4 kb. Sequencing of clone q10 yielded a sequence having an open reading frame, in which a signal peptide, an extracellular domain, a transmembrane domain and part of the intracellular domain were identified (Figure 2). DNA sequences coding for the N-terminal protein sequence, as well as the sequences of the three CNBr peptides cb3, cb6 and cb7 of the urinary IFNAB-BP were identified within the extracellular domain coded by the DNA of clone q10. Some Cys and Ser residues (dot underlined, Figure 2) were not correctly identified by the protein sequencing. However, it is known that the method used for protein sequencing does not call Cys residues and occasionally it misses Ser residues. Also an Asn residue in peptide cb6 was not detected, indicating that it is glycosylated. Comparison of the DNA sequence of clone q10 with Genebank database did not show any identity with any known sequence. Hence this clone contains a new DNA sequence.

### Example 5. Northern blotting of human mRNA

A radiolabelled DNA probe was prepared from clone q10 and used for Northern blot hybridization of poly A+ mRNA from two human cell lines: Daudi and WISH. In both cases two specific bands were observed, one, corresponding to 1.5 kb and another one, corresponding to 4.5 kb. Based on the intensity of the bands it was estimated that the 1.5 kb mRNA is about twice as abundant as the 4.5 kb mRNA. The signal obtained with the RNA from WISH cells was barely seen, while the signal of RNA from Daudi cells (Figure 3) was detectable. The 1.5 kb mRNA is translated into a precursor of IFNAB-BPI which is a cell surface interferon receptor. The longer mRNA represents a different transcript, coding for a different protein that shares at least about 100 amino acid residues with IFNAB-BPI. This protein is the precursor of IFNAB-BPII, later shown to be a soluble form of the interferon-α/β receptor.

### Example 6. Identification of complete cDNA clones of IFNAB-BPI and IFNAB-BPII

A human monocyte cDNA library, constructed in phage λpCEV9 (Gutkind, J. S., et al., Molec. Cell. Biol. *11*, 1500-1507, 1991), was then screened with a 397 bp probe made by PCR from the coding region of clone q10. We isolated 22 clones with a 1.5 kb insert and two clones with a 4.5 kb insert from 10⁶ independent phages. DNA sequence analysis of two 1.5 kb clones (λpCEV9-m6 and λpCEV9-m24), as well as the entire open reading frame of the two 4.5 kb clones (λpCEV9-m19 and λpCEV9-m27) was performed. The 1.5 kb clones coded for a complete precursor of IFNAB-BPI, which is a cell surface receptor, with an open reading frame of 331 codons (Figure 4). The protein and CNBr peptide sequences, obtained from urinary IFNAB-BP (dot underlined, Figure 4), were all identified within the translated DNA sequence. Partial sequencing of the two 4.5 kb clones revealed the same 5' sequence of 237 codons as present in the 1.5 kb clones, followed by a different sequence that included a termination signal after codon 239 (Figure 5). Altogether, the following codons were different: codon 13 (Leu instead of His); codon 108 (Thr instead of Ile) and codons 238-240 (Phe-Ser-Stop instead of Ser-Ala-Ser). No open reading frame was seen beyond the stop codon in any of the three reading frames in both of the 4.5 kb clones. Hence the 4.5 kb cDNA codes for a precursor of a truncated soluble receptor which is IFNAB-BPII, identical in its C-terminal sequence to the one isolated from urine. The two mRNAs coding for the precursor proteins of both IFNAB-BPI and IFNAB-BPII are derived from the same gene, probably by alternative splicing.

### Example 7. Construction of a mammalian expression vector and production of recombinant soluble IFNAB-BPI and IFNAB-BPII

A DNA coding for the signal sequence and the extracellular domain of IFNAB-BPI was generated by PCR with VENT DNA polymerase (Stratagene), using synthetic sense and antisense primers, carrying Xba I restriction sites (Figure 6A) and using q10 DNA as a template. The resulting PCR product (Figure 6B) was restricted by Xba I and ligated into the expression vector pEF-BOS to yield pEF-BOS-IFNAB-BPI (Figure 6C, reference 46). The construct was confirmed by DNA sequencing. Competent E. Coli were transformed and clones having the IFNAB-BPI sequence in a correct orientation were isolated. The pEF-BOS-sABR construct was used for transfection of monkey COS cells. These cells expressed 12 ng/ml of recombinant soluble IFNAB-BPI that was obtained in the cell culture supernatant, as determined by ELISA and by its ability to inhibit the biological (antiviral) activity of human interferons alpha and beta. In analogy, the DNA region coding for IFNAB-BPII in the 4.5 kb clone is inserted into a mammalian expression vector as described for the extracellular domain of IFNAB-BPI and used for transforming cells. Such cells are producing active IFNAB-BPII that is secreted to the culture medium of said cells.

### Example 8. Construction of eukaryotic expression vectors and expression of IFNAB-BPI and IFNAR in murine cells

A DNA coding for the entire of IFNAB-BPI was generated by PCR with VENT DNA polymerase (Stratagene), using synthetic sense and antisense primers, carrying Xba I restriction sites and using plasmid pCEV9-m6 as a template. The resulting PCR product was restricted by Xba I and ligated into the expression vector pEF-BOS to yield pEF-BOS-IFNABR. The cDNA corresponding to IFNAR (14) was generated by RT-PCR (48), using specific oligonucleotides. The amplified product was cloned into the Xba I restriction site of the pEF-BOS expression vector (46), to yield pEF-BOS-IFNAR. These construct were confirmed by DNA sequencing. Competent E. Coli were transformed and clones having the IFNAB-BPI and IFNAR sequences in a correct orientation were isolated.

Murine cells expressing the cloned IFNAB-BPI cDNA in a stable manner were developed. Exponentially growing NIH-3T3 cells (1.5x10⁶ in 10 cm plates ) were cotransfected by the calcium phosphate precipitation method (49) with pSV2neo (2 µg), together with pEF-BOS-IFNABR (10 µg DNA). Independent G418-resistant colonies were identified and sub-cloned. Clones expressing high levels of IFNAB-BPI were identified by binding of an antibody directed against the urinary IFNAB-BPI and by binding of ¹²⁵I-IFN-α2 (Table IV).

For binding of anti IFNAB-BPII antibodies, cells (1x10⁶) were seeded in 35 mm wells (6 well plates, Costar) and grown to confluency (20 hr). The cells were washed with DMEM containing 2% FBS and 0.1% sodium azide (Wash medium) followed by an incubation of 20 min. with the Wash medium. Rabbit anti IFNAB-BPII antibodies (2 ml, 1:500 in the Wash medium) were added to the washed wells and the cells were incubated for 2h at room temperature. The cells were washed 3 times, ¹²⁵I-protein A (2 ml, 250,000 cpm in the Wash medium) was added and the cells were further incubated for 45 min. The cells were washed 3 times, harvested with trypsin and counted.

For binding of ¹²⁵I-IFN-α2, cells (1x10⁶) were seeded in 35 mm wells (6 well plates, Costar) and grown to confluency (20 hr). The cells were washed with DMEM containing 2% FBS and 0.1% sodium azide (Wash medium) followed by an incubation of 20 min. with the Wash medium. ¹²⁵I-IFN-α2 (2-3x10⁵ cpm, 10⁸ units/mg, 5x10⁷ cpm/µg) was added and incubation continued for 2 hr at room temperature. The cells were washed 3 times, harvested with trypsin and counted.

SDS-PAGE under non-reducing conditions of a detergent extracts of positive clones (e.g., Clone 369.11), followed by immunoblotting with the above mentioned antibody gave a strong band of about 51 kDa (Figure 7).

Murine cells expressing IFNAR were similarly developed by transfection with plasmid pEF-BOS-IFNAR. Clone No. 470.6 was IFNAR-positive, as determined by the ability of huIFN-αB to effectively induce an antiviral response in these cells. As expected, other type I IFNs (e.g., huIFN-β) were not active in Clone 470.6.

Clones 369.11 and 470.6, expressing IFNAB-BPI and IFNAR, were then transfected with the complementary receptor protein (pEF-BOS-IFNAR and pEF-BOS-IFNABR, respectively). For stable co-expression, G418-resistant clones, expressing either IFNAR or IFNAB-BPI were transfected with pSV2hygro (2 µg), together with either pEF-BOS-IFNABR or pEF-BOS-IFNAR as above. Hygromycin and G418-resistant clones, co-expressing both IFNAR and IFNAB-BPI were isolated and sub-cloned. IFNAR-positive clones, derived from Clone 369.11, were identified by their antiviral response to huIFN-αB, while IFNAB-BPI-positive clones, derived from Clone 470.6, were identified by binding of both anti IFNAB-BPII antibodies and ¹²⁵I-IFN-α2. Clone 508.12, derived from Clone 369.11, and Clone 1306, derived from Clone 470.6, bound both ¹²⁵I-IFN-α2 and IFN-α/βR antibody (Table IV). In addition, they responded to huIFN-αB in an antiviral assay. Hence we concluded that these clones express both IFNAB-BPI and a functional IFNAR.

**Table IV.**

| Expression of IFNAB-BPI in various cells. | | |
|---|---|---|
| Cells | Bound IFNAB-BPII antibody (cpm) | Bound ¹²⁵I-IFN-α2 (cpm) |
| 470.6 (IFNAR) | 236 | 90 |
| 369.11 (IFNAB-BPI) | 4243 | 12,500 |
| 508.12 (IFNAB-BPI & IFNAR)^{a} | 7728 | 70,240 |
| 1306 (IFNAR & IFNAB-BPI)^{b} | 3369 | 32,000 |

| | | |
|---|---|---|
| ^{a} Derived from Clone 369.11 | | |
| ^{b} Derived from Clone 470.6 | | |

### Example 9. Determination of the affinity of IFNAB-BPI and IFNAR expressed in murine cells

Clones expressing either IFNAR or IFNAB-BPI were tested for binding of ¹²⁵I-huIFN-α2 and the binding data were evaluated by a Scatchard analysis. Cells (1x10⁶) were seeded in 35 mm wells (6 well plates, Costar) and grown to confluency (20 h). The cells were washed with DMEM containing 2% FBS and 0.1% sodium azide (Wash medium) followed by an incubation of 20 min. with the same medium. ¹²⁵I-IFN-α2 (2-3x10⁵ cpm, 10⁸ units/mg, 5x10⁷ cpm/µg) was added, together with the indicated concentrations of non-labeled IFN-α2 and incubation continued for 2 hr at room temperature. The cells were washed 3 times with the Wash Medium, harvested with trypsin and counted. Binding data was analyzed by the LIGAND program (50).

Cells expressing IFNAR only (Clone 470.6) did not exhibit any specific binding of ¹²⁵I-IFN-α2 (Figure 8 A) and hence, no *Kd* value of such putative binding sites could be derived. In contrast with Clone 470.6, high affinity, specific and saturable binding was obtained with cells expressing IFNAB-BPI alone (Clone 369.11). The *Kd* of this binding was 3.6x10⁻⁹ M at 23° C (Table V).

Binding of ¹²⁵I-IFN-α2 to 508.12 cells (expressing both IFNAB-BPI and IFNAR) was evaluated by a Scatchard analysis and the results were compared with those of Clone 369.11 (expressing only IFNAB-BPI). Upon co-expression of IFNAB-BPI and IFNAR, a saturable binding was obtained and the affinity for IFN-α2 increased by about 10 fold (Figure 8), approaching that of the receptor in Daudi cells (*Kd* = 4.0x10⁻¹⁰ M vs. 1.6x10⁻¹⁰ M, respectively, Table V). This result indicates that IFNAR and IFNAB-BPI cooperate in ligand binding.

**Table V.**

| Binding characteristics of various host cells (ligand = ¹²⁵I-IFN-α2). | | |
|---|---|---|
| Cell (receptor) | binding sites per cell | *Kd* (M) at 20°C |
| human Daudi | 4900±11% | 1.6x10⁻¹⁰ |
| 470.6 (IFNAR) | 0 | (-) |
| 369.11 (IFNAB-BPI) | 80,000±11% | 3.6x10⁻⁹ |
| 508.12 (IFNAB-BPI & IFNAR) | 59,000±10% | 4.07x10⁻¹⁰ |

### Example 10. Determination of the affinity of urinary IFNAB-BPII

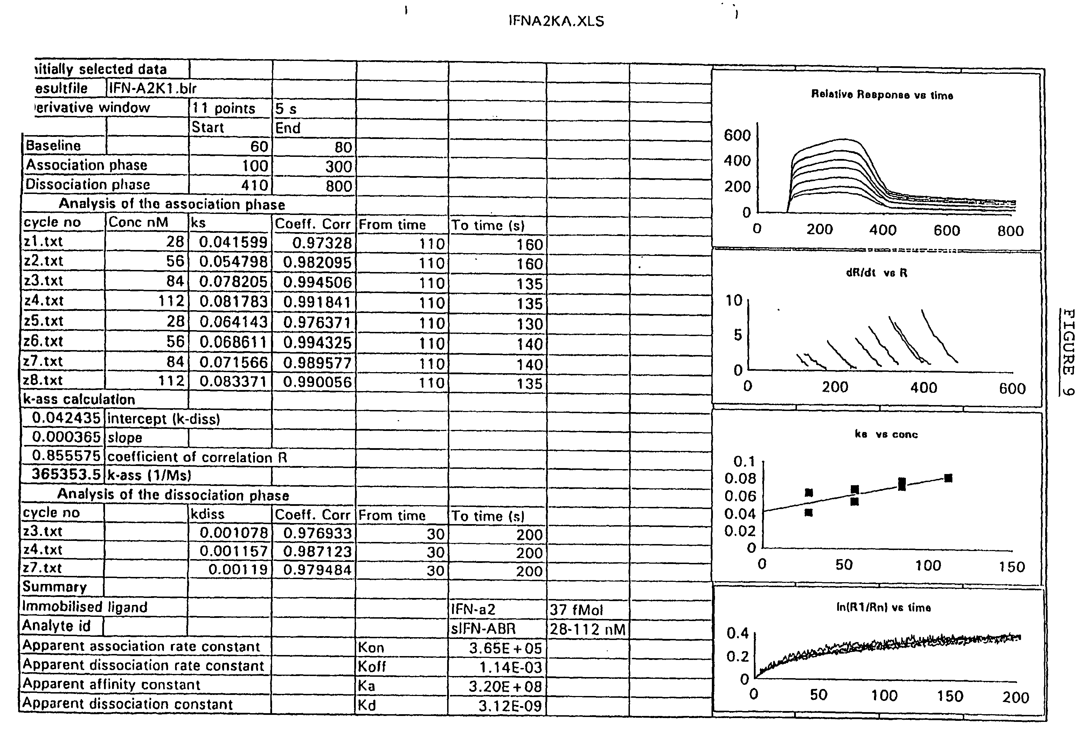

Human IFN-α2 was immobilized on a sensor chip of BIAcore (Pharmacia, Sweden) by an automatic procedure provided by the manufacturer. About 30 fmol of IFN-α2 were immobilized. Urinary IFNAB-BPII diluted in phosphate-buffered saline (PBS) to several concentrations (28-112 nM) was passed through the sensogram chip and the extent of association and dissociation (in PBS) was recorded. Based on the resulting data a *Kd* value of 3.12x10⁻⁹ M was calculated (Figure 9). Thus the affinity of urinary IFNAB-BPII is very similar to that of IFNAB-BPI expressed in host cells.

### Example 11. Expression of IFNAB-BPI and IFNAB-BPII in E.Coli, yeast and insect cells.

IFNAB-BPI and IFNAB-BPII are also produced by additional recombinant cells such as prokaryotic cells, e.g., E.Coli, or other eukaryotic cells, such as yeast and insect cells. Well known methods are available for constructing appropriate vectors, carrying DNA that codes for either IFNAB-BPI or IFNAB-BPII and their active fractions and suitable for transforming E.Coli and yeast cells, or infecting insect cells in order to produce recombinant IFNAB-BPI and IFNAB-BPII. For expression in yeast cells, the DNA coding for IFNAB-BPI or IFNAB-BPII (Examples 5 and 6) is cut out and inserted into expression vectors suitable for transfection of yeast cells. For expression in insect cells, the DNA coding for IFNAB-BPI or IFNAB-BPII is inserted into baculovirus and the insect cells are infected with said recombinant baculovirus. For expression in E.Coli the DNA coding for either IFNAB-BPI or IFNAB-BPII is subjected to site directed mutagenesis with appropriate oligonucleotides, so that an initiation ATG codon is inserted just prior to the first codon of mature IFNAB-BPI (Figure 2) or IFNAB-BPII. Alternatively, such DNA can be prepared by PCR with suitable sense and antisense primers. The resulting cDNA constructs are then inserted into appropriately constructed prokaryotic expression vectors by techniques well known in the art (35).

### Example 12: Construction of recombinant fusion proteins of IFNAB-BPI and IFNAB-BPII

The production of proteins comprising either the ligand-binding domain of IFNAB-BPI or IFNAB-BPII, fused to the constant region of IgG1 heavy chain may be carried out as follows: the DNA of IFNAB-BPI or IFNAB-BPII is subjected to site-directed mutagenesis with appropriate oligonucleotides so that a unique restriction site is introduced immediately before and after sequences coding to the ligand binding extracellular domains. Alternatively, such DNA may be prepared by PCR with specifically designed primers bearing the restriction sites. Another plasmid bearing the constant region of IgG1 heavy chain, e.g. pRKCO42Fc1(47) is subjected to similar site-directed mutagenesis to introduce the same unique restriction site as close as possible to Asp 216 of IgG1 heavy chain in a way that allows translation in phase of the fused protein. A dsDNA fragment, consisting of 5' non-translated sequences and encoding for either IFNAB-BPII or the ligand-binding domain of IFNAB-BPI is prepared by digestion at the unique restriction sites. The mutated pRKCD42Fc1 is similarly digested to generate a large fragment containing the plasmid and the IgG1 sequences. The two fragments are then ligated to generate a new plasmid, encoding a polypeptide precursor consisting of either IFNAB-BPII or the ligand-binding domain of IFNAB-BPI and about 227 C-terminal amino acids of IgG1 heavy chain (hinge region and CH2 and CH3 domains). The DNA encoding the fused proteins may be isolated from the plasmid by digestion with appropriate restriction enzymes and then inserted into efficient prokaryotic or eukaryotic expression vectors.

### Example 13: Construction of recombinant fusion proteins of IFNAB-BPI and IFNAB-BPII together with IFNAR

The production of proteins comprising either the extracellular domain of IFNAB-BPI or IFNAB-BPII, fused to the constant region of IgG1 heavy chain may be carried out as described in example 12. The production of a protein comprising the extracellular domain of IFNAR, fused to the constant region of IgG1 light chain is similarly carried out. Eukaryotic expression vectors coding for either the ligand-binding domain of IFNAB-BPI fused to the constant region of IgG1 heavy chain or IFNAB-BPII, fused to the constant region of IgG1 heavy chain are used for co-transfection of suitable mammalian host cells together with an eukaryotic expression vector coding for the extracellular domain of IFNAR, fused to the constant region of IgG1 light chain. Positive transfectants will secrete a composite protein consisting of the IgG1 constant regions, the extracellular domain of IFNAR replacing the variable regions of IgG1 light chains and either IFNAB-BPII or the ligand-binding domain of IFNAB-BPI replacing the variable regions of IgG1 heavy chains.

In another example the constant regions of the heavy and light chains are switched, namely the extracellular domain of IFNAR is fused to the constant regions of IgG2 heavy chain while either the ligand-binding domain of IFNAB-BPI or IFNAB-BPII are fused to the constant regions of IgG2 light chain.

Based on example 9, these fused proteins are expected to exhibit about 10-fold higher affinity for IFN-α as compared with that of IFNAB-BPI or IFNAB-BPII.

### Reference Example 14: Preparation of polyclonal antibodies to IFNAB-BP

Rabbits were initially injected subcutaneously with 5 µg of a pure preparation of the urinary IFNAB-BP emulsified in complete Freund's adjuvant. Three weeks later they were injected again subcutaneously with 5 µg of the preparation in incomplete Fruend's adjuvant. Four additional injections as solution in PBS were given at 10 day intervals. The rabbits were bled 10 days after the last immunization. The development of antibody level was followed by a solid-phase radioimmunoassay (sRIA), using 96-well PVC plates coated overnight at 4°C with IFNAB-BP (1 µg/ml), in phosphate-buffered saline (PBS). The plates were then blocked with bovine serum albumin (BSA, 0.5%), Tween 20 (Sigma USA, 0.05%) in PBS overnight at 4°C. The plates were reacted with 5 fold dilutions of the rabbit antiserum for 4 hours at room temperature, washed and! reacted with ¹²⁵I-protein A (10⁵ cpm/well) in PBS for 45 min. at room temperature. The plates were then washed; individual wells were cut and counted. The titer is calculated as the reciprocal of the maximal dilution that gave counts 10 fold higher than control antiserum. The titer after 5 injections was greater than 1:60,000.

The development of antibody level was also followed by the ability of the antiserum to block the antiviral activity of human IFN-α2. Preformed monolayers of human WISH cells in 96-well plates were incubated with two-fold dilutions of the antiserum, starting at a dilution of 1:250 in well no. 1, for 1 hour at 37°C. IFN-α2 (10 u/ml, final) was then added and after 1 hour at 37°C, the cells were challenged with vesicular stomatitis virus. The neutralizing titer after 7 immunizations was 120,000 antiviral u/ml.

### Reference EXAMPLE 15: Preparation of monoclonal antibodies to IFNAB-BP

Female Balb/C mice (3 months old) were first injected with 2 µg purified IFNAB-BP in an emulsion of complete Freund's adjuvant, and three weeks later, subcutaneously in incomplete Freund's adjuvant. Three additional injections were given at 10 day intervals, subcutaneously in PBS. A binding titer of 1:60,000 was obtained by sRIA (see Example 9). Final boosts were given intraperitoneally 4 and 3 days before the fusion to the mouse showing the highest binding titer. Fusion was performed using NSO/1 myeloma cell line and lymphocytes prepared from both the spleen and lymph nodes of the animal as fusion partners. The fused cells were distributed into microculture plates and the hybridomas were selected in DMEM supplemented with HAT and 15% horse serum. Hybridomas that were found to produce antibodies to IFNAB-BP were subcloned by the limiting dilution method and injected into Balb/C mice that had been primed with pristane for the production of ascites. The isotypes of the antibodies were defined with the use of a commercially available ELISA kit (Amersham, UK).

The screening of hybridomas producing anti-IFNAB-BP monoclonal antibodies was performed as follows: Hybridoma supernatants were tested for the presence of anti-IFNAB-BP antibodies by an inverted solid phase radioimmunoassay (IRIA). PVC microtiter plates (Dynatech Laboratories, Alexandria, VA) were coated with affinity purified goat anti-mouse serum F(ab)₂ antibodies (Jackson Labs, USA) (10 µg/ml, 100 µl/well). Following overnight incubation at 4°C the plates were washed twice with PBS containing BSA (0.5%) and Tween 20 (0.05%) and blocked in washing solution for at least 2 hr. at 37°C. Hybridoma culture supernatants (100 µl/well) were added and the plates were incubated for 4 hr. at 37°C. The plates were then washed three times with the washing solution and ¹²⁵I-IFNAB-BP (100 µl, 10⁵ cpm) was added for further incubation of 16 hr. at 4°C. The plates were washed 3 times and individual wells were cut and counted in a gamma counter. Samples giving counts that were at least 5 times higher than the negative control value were considered positive (Table VI). Five positive clones were selected, subcloned for further studies and characterized. All clones were of IgG₁ isotype.

**Table VI**

| **Clones producing monoclonal antibodies to IFNAB-BP** | | |
|---|---|---|
| **Clone No.** | **Dilution** | **IRIA - CPM** |
| 2.1 | 1:1 | 2,140 |
| 5.73 | 1:1 | 3,292 |
| 30.24 | 1:1 | 5,548 |
| 46.10 | 1:1000 | 29,818 |
| 70.6 | 1:1 | 1,214 |
| Control antibody | 1:1 | 20 |

### Reference EXAMPLE 16: Affinity chromatography of IFNAB-BP with monoclonal antibodies

Antibodies against IFNAB-BP were utilized for the purification of IFNAB-BP by affinity chromatography. The monoclonal antibody No. 5.73 was used in this example for affinity chromatography. Ascitic fluid containing the monoclonal antibody secreted by hybridoma No. 5.73 was purified by ammonium sulfate precipitation at 50% saturation followed by extensive dialysis against PBS. About 10 mg of immunoglobulins were bound to 1 ml Affigel 10 (Bio-Rad USA), as specified by the manufacturer.

250 ml of human urinary proteins (equivalent to 250 1 of crude urine) were loaded on 0.5 ml of the anti IFNAB-BP antibody column at 4°C at a flow rate of 0.25 ml/min. The column was washed with PBS until no protein was detected in the washings. IFNAB-BP was eluted by 25 mM citric acid buffer, pH 2.2 (8 x 1 column volume fractions) and immediately neutralized by 1 M Na₂CO₃. Silver stain analysis of SDS PAGE of the eluted fractions reveals a major band of MW of 40,000. Further purification of this preparation was obtained by size exclusion chromatography.

### Reference EXAMPLE 17: ELISA test of IFNAB-BPII

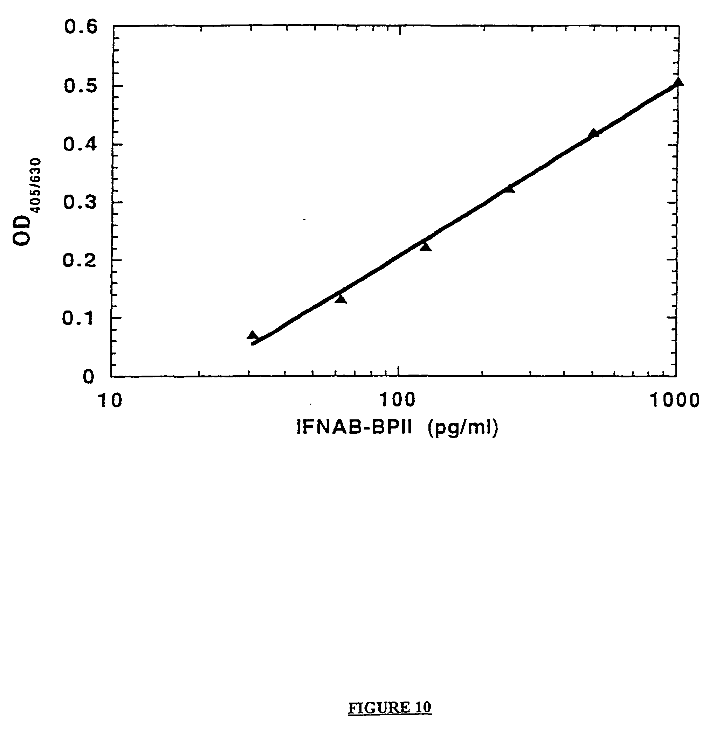

Microtiter plates (Dynatech or Maxisorb, by Nunc) were coated with anti-IFNAB-BP monoclonal antibody No. 46.10 (Ig fraction, 120 µl/well, 10 µg/ml in PBS) overnight at 4°C. The plates were washed with PBS containing BSA (0.5%), Tween 20 (0.05%) and NaN₃ (0.02%) (Blocking Solution) and blocked in the same solution overnight at 37°C. The tested samples were serially diluted twofold (starting with 1:4) in the Blocking Solution containing 0.1% NP40 and 0.65 M NaCl and added to the wells (100 µl/well) for 4 hr. at 37°C. The plates were then washed 3 times with PBS containing 0.05% Tween 20 (PBS/Tween) followed by the addition of rabbit anti-IFNAB-BPII serum (1:1000 in Blocking Solution but without NaN₃, 100 µl/well) for further incubation overnight at 4°C. The plates were washed 3 times with PBS/Tween, (100 µl/well), and a conjugate of goat-anti-rabbit horseradish peroxidase (HRP, Jackson Labs, 1:10,000 in PBS/Tween, 100 µl/well) was added for 2 hr. at room temperature. The plates were washed 3 times with PBS/Tween and the color was developed by adding to each well 100 µl of a freshly prepared solution of ABTS (2,2'-azino-bis (3-ethylbenzthiazoline-6-sulfonic acid, Sigma, 10 mg; 6.4 ml H₂O; 2.2 ml of 0.2M Na₂HPO₄; 1.4 ml 0.2 M citric acid; 1 µl H₂O₂) as a substrate. Color develops by 30 min. and the reaction may be stopped by addition of 100 µl/well of 0.2 M citric acid. The plates were read by an automatic ELISA reader at 405 nm, correcting for non-specific reading at 630 nm. The lower limit of detection of this assay was 30 pg/ml (Figure 10).

The foregoing description of the specific embodiments reveal the general nature of the invention so that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

### References

1. Taylor, J.L. and Grossberg, S.E. (1990). Recent progress in interferon research: molecular mechanisms of regulation, action and virus circumvention. Virus Research 15:1-26.
2. Bisceglie A.M., Martin, P., Kassianides, C., Lisker-Melman, M., Murray, L., Waggoner, J., Goodmann, Z., Banks, M.S. and Hoofnagle, J.H. (1989). Recombinant interferon alpha therapy for chronic hepatitis C. A randomized, double-bind, placebo-controlled trial. New Eng. J. Med. 321:1506-1510.
3. McDonnell, W.M. and Elta G.H. (1992). Acute hepatitis C infection: interferon finally succeeds. Gastroenterology (US) 103:1359-1360.
4. Friedman-Kien, A.E., Eron, L.J., Conant, M., Growdon, W., Badiak, H., Bradstreet, P.W., Fedorczyk, D., Trout, R. and Plesse, T.F. (1988). Natural interferon alpha for treatment of condylomata acuminata. J. Am. Med. Assn., 259: 533-538.
5. Mains, J. and Handley, J. (1992). Interferon: current and future clinical uses in infectious disease practice. Int. J. Stud. AIDS 3:4-9.
6. Berman, E., Heller, G., Kempin, S., Gee, T., Tran, L. and Clarkson, B. (1990). Incidence of response and long term follow up in patients with hairy cell leukemia treated with recombinant interferon Alpha-2a, Blood 75: 839-845.
7. Talpaz M., Kantarjian, H.M., McCredie, K.B., Keating, M.J., Trujillo, J. and Gutterman, J. (1987), Clinical investigation of human alpha interferon in chronic myelogenous leukemia. Blood 69:1280-1288.
8. De Wit, R., Schattenkerk, J.K.M.E., Boucher, C.A.B., Bakker, P.J.M., Veenhof, K.H.N. and Danner, S.A. (1988). Clinical and virological effects of high-dose recombinant-interferon-a in disseminated AIDS-related Kaposi's sarcoma. Lancet 2: 1214-1222.
9. Klippel, J.H., Carrete, S., Preble, D.T., Friedman, R.M. and Grimley P.M. (1985). Serum alpha interferon and lymphocyte inclusions in systemic lupus erythematosus. Annals of the Rheumatic Diseases 44:104-108.
10. Lau, A.S., Der, S.D., Read, S.E., and Williams, B.R. (1991). Regulation of tumor necrosis factor receptor expression by acid-labile interferon-alpha from AIDS sera. AIDS Res. Hum. Retroviruses 7:545-552
11. Stewart, T.A. (1993). Induction of type I diabetes by interferon-a in transgenic mice. Science 260:1942-1946.
12. Tsavaris, N., Mylonakis, N., Bacoyiannis, C., Tsoutsos, H., Karabelis, A., and Kosmidis, P. (1993). Treatment of renal cell carcinoma with escalating doses of alpha-interferon. Chemotherapy (Switzerland) 39:361-366.
13. Branka, A.A. and Baglioni, C. (1981). Evidence that types I and II interferons have different receptors. Nature 294:768-770.
14. Uze G., Lutfalla, G. and Gresser, I. (1990). Genetic transfer of a functional human interferon a receptor into mouse cells: cloning and expression of its cDNA. Cell 60: 225-234.
15. Colamonici, O.R. et al. (1990). Characterization of three monoclonal antibodies that recognize the interferon-a2 receptor. Proc. Natl. Acad. Sci. USA 87:7230-7234.
16. Platanias, L.C. et al. (1992). Expression of the IFN-a receptor in hairy cell leukemia, Brit. J. Haematology 82:541-546.
17. Colaminici, O.R. et al. (1993). Identification of a novel subunit of the type I Interferon receptor localized to human chromosome 21. J. Biol. Chem. 268:10895-10899.
18. Benoit, P. et al. (1993). A monoclonal antibody to recombinant human IFN-a receptor inhibits biologic activity of several species of human IFN-a, IFN-b and IFN-omega. J. Immunol. 150:707-716.
19. Novick, D., Engelmann, H., Wallach, D. and Rubinstein, M. (1989). Soluble cytokine receptors are present in normal human urine. J. Exp. Med. 170: 1409-1414.
20. Novick, D., Engelmann, H., Wallach, D., Leitner, O., Revel, M. and Rubinstein, M. (1990). Purification of soluble cytokine- receptors from normal human urine by ligand-affinity and immune-affinity chromatography. J. Chromatography 510: 331-337.
21. Novick, D., Engelmann, H., Revel, M., Leitner, O. and Rubinstein M. (1991). Monoclonal antibodies to the soluble IL-6 receptor: affinity purification, ELISA, and inhibition of ligand binding. Hybridoma 10:137-146.
22. Engelmann, H., Novick, D. and Wallach D. (1990). Two tumor-necrosis-factor binding proteins purified from human urine. Evidence for immunological cross reactivity with cell surface tumor-necrosis-factor receptors. J. Biol. Chem. 265: 1531-1536.
23. Engelmann, H., Aderka, D., Rubinstein, M., Rotman, D. and Wallach, D. (1989). A tumor necrosis factor-binding protein purified to homogeneity from human urine protects cells from tumor necrosis factor toxicity. J. Biol. Chem. 264:11974-11980.
24. Seckinger, P., Isaaz, S. and Dayer, J.M. (1988) A human inhibitor of tumor necrosis factor alpha. J. Exp. Med. 167:1511-1516.
25. Maliszewski, C.R. and Fanslow, W.C. (1990). Soluble receptors for IL-1 and IL-4. Biological activity and therapeutic potential. Trends in Biotechnol. 8:324-329.
26. Eastgate, J.A., Symons, J.A. and Duff G.W. (1990). Identification of an interleukin-1 beta binding protein in human plasma. FEBS Letters 260:213-216.
27. Fanslow, F.W., Sims, J.E., Sasenfeld, H., Morrisey, P.J., Gillis, S., Dower, S.K. and Widmer, M.B. (1990). Regulation of alloreactivity in vivo by soluble form of the interleukin-1 receptor. Science 248:739-742.
28. Mosley, B., Beckman, M.P., March, C., J., Idzerda, R.L., Gimpel, S., D., VandenBos, T., Friend, D., Alpert, A., Anderson, D., Jackson, J., Wignall, J.M. Smith C., Gallis, B., Sims, J.E., Urdal, D., Widmer, M.B., Cosman, D. and Pari, L.S. (1989). The murine interleukin-4 receptor: molecular cloning and characterization of secreted and membrane forms. Cell 59:335-348.
29. Marcon, L., Fritz, M.E., Kurman, C.C., Jensen, J.C. and Nelson, D.L. (1988). Soluble TAC peptide is present in the urine of normal individuals and at elevated levels in patients with adult T-cell leukemia. Clin. Exp. Immunol. 73:29-33.
30. Josimovic-Alasevic, O., Hermann, T. and Diamanstein, T. (1988). Demonstration of two distinct forms of released low-affinity type interleukin-2 receptors. Eur. J. Immunol. 18:1855-1857.
31. Goodwin, R.G., Friend, D., Ziegler, S.F., March. C.J., Namen, A.E. and Park, L.S. (1990). Cloning of the human and murine interleukin-7 receptors: demonstration of a soluble form and homology to a new receptor superfamily. Cell 60:941-951.
32. Novick, D., Cohen, B. and Rubinstein, M. (1992). Soluble IFN-a receptor molecules are present in Body Fluids, FEBS Letters, 314:445-448.
33. Pearson, W.R. and Lipman, D.G. (1988), Improved tools for biological sequence comparison, Proc. Natl. Acad. Sci. USA, 85:2444-2448.
34. Okayama, H. and Berg, P. (1983) AcDNA cloning vector that permits expression of cDNA inserts in mammalian cells. Mol. Cell. Biol. 3:280-289.
35. Maniatis et al., Molecular Cloning: A Laboratory Manual, Cold Spring Harbor Laboratory, New York, 1982
36. Gryczan, T., "The Molecular Biology of the Bacilli", Academic Press, NY (1982), pp. 307-329).
37. Kendall, K.J. et al. (1987) J. Bacteriol. 169:4177-4183).
38. Chater, KF. et al., in "Sixth International Symposium on Actinomycetales Biology", Akademiai Kaido, Budapest, Hungary (1986), pp. 45-54).
39. John, J.F., et al. (1986) Rev. Infect. Dis. 8:693-704).
40. Izaki, K. (1978) Jpn. J. Bacteriol. 33:729-742).
41. Botstein, D., et al. (1982) Miami Wint. Symp. 19:265-274.
42. Broach, JR., in "The Molecular Biology of the Yeast Saccharomyces: Life Cycle and Inheritance", Cold Spring Harbor Laboratory, Cold Spring Harbor, NY, pp. 445-470 (1981).
43. Broach, J.R., (1982) Cell 28:203-204
44. Bollon, D.P., et al. (1980) J. Clin. Hematol. Oncol. 10:39-48.
45. Maniatis, T., in "Cell Biology: A Comprehensive Treatise, Vol. 3: Gene Expression", Academic Press, NY, pp. 563-608 (1980)).
46. Mizushima, S. and Nagata, S. (1990) pEF-BOS, a powerful mammalian expression vector. Nucleic Acid Res. 18:5322-5328.
47. Byrn R.A. et al., 1990, Nature (London) 344:667-670
48. Frohman, M.A., Dush, M.K. and Martin, G.R. (1988) *Proc. Nat'l*. *Acad. Sci. USA,* 85, 8998-9002.
49. Graham, F.L. and Van der Eb, A.J. (1973) *Virology,* 52, 456-467.
50. Munson, P.J. and Rodbard, D. (1980) *Anal*. *Biochem*., 107, 220-239.
51. Yamada, T. et al., Neurosci.Lett., 181: 61-64 (1994)

## Claims

1. A DNA molecule encoding an IFN-α/β binding protein selected from
(a) IFNAB-BPI having the amino acid sequence shown in Fig. 2;
(b) IFNAB-BPII having the amino acid sequence shown in Fig. 5; and
(c) precursors or fusion proteins of the proteins of (a) or (b).

2. An mRNA molecule being the product of transcription of the DNA molecule of claim 1.

3. The mRNA molecule according to claim 2 selected from
(a) an mRNA molecule of about 1.5 kb which, when translated, provides an IFNAB-BPI precursor; and
(b) an mRNA molecule of about 4.5 kb which, when translated, provides an IFNAB-BPII.

4. A replicable expression vehicle comprising the DNA molecule according to claim 1.

5. The replicable expression vehicle according to claim 4 which is the plasmid pEF-BOS-IFNAB-BPI, the construction of which is described in Example 7.

6. A host cell transformed with the replicable expression vector of claim 4 or 5.

7. The host cell according to claim 6 which is a prokaryotic cell.

8. The host cell according to claim 6 which is a eukaryotic cell.

9. The host cell according to claim 8 which is a monkey COS cell.

10. An IFN-α/β binding protein encoded by the DNA sequence of claim 1 or the RNA sequence of claim 2 or 3 or which is produced by the host cell of any one of claims 6 to 9, or a salt of said binding protein.

11. The IFN-α/β binding protein according to claim 10 which is a mature protein or a salt thereof.

12. A pharmaceutical composition comprising the IFN-α/β binding protein according to claim 10 or 11 or a salt thereof, optionally in combination with a pharmaceutically acceptable carrier.

13. Use of the IFN-α/β binding protein according to claim 10 or 11 for the preparation of a pharmaceutical composition for the modulation of IFN-α or IFN-β activity.

14. Use of the IFN-α/β binding protein according to claim 10 or 11 for the preparation of a pharmaceutical composition for the inhibition of IFN-α or IFN-β activity.

15. A process for the production of the IFN-α/β binding protein according to claim 10 or 11, comprising culturing the host cell according to any one of claims 6 to 9 and recovering said IFN-α/β binding protein.

16. A diagnostic composition comprising the DNA molecule of claim 1 and/or the IFN-α/β binding protein according to claim 10 or 11.

## Patentansprüche

1. DNA-Molekül, das ein IFN-α/β-Bindeprotein codiert, ausgewählt aus
(a) IFNAB-BPI, das die in Figur 2 gezeigte Aminosäuresequenz hat;
(b) IFNAB-BPII, das die in Figur 5 gezeigte Aminosäuresequenz hat; und
(c) Vorläufern oder Fusionsproteinen der Proteine nach (a) oder (b).

2. mRNA-Molekül, das das Produkt der Transkription des DNA-Moleküls nach Anspruch 1 ist.

3. mRNA-Molekül nach Anspruch 2, ausgewählt aus
(a) einem mRNA-Molekül von etwa 1,5 kb, welches, wenn es translatiert wird, einen IFNAB-BPI-Vorläufer liefert; und
(b) einem mRNA-Molekül von etwa 4,5 kb, welches, wenn es translatiert wird, ein IFNAB-BPII liefert.

4. Replizierbares Expressionsvehikel, umfassend das DNA-Molekül nach Anspruch 1.

5. Replizierbares Expressionsvehikel nach Anspruch 4, welches das Plasmid pEF-BOS-IFNAB-BPI ist, dessen Konstruktion in Beispiel 7 beschrieben ist.

6. Wirtszelle, die transformiert ist mit dem replizierbaren Expressionsvektor nach Anspruch 4 oder 5.

7. Wirtszelle nach Anspruch 6, die eine prokaryontische Zelle ist.

8. Wirtszelle nach Anspruch 6, die eine eukaryontische Zelle ist.

9. Wirtszelle nach Anspruch 8, die eine Affen-COS-Zelle ist.

10. IFN-α/β-Bindeprotein, das durch die DNA-Sequenz nach Anspruch 1 oder die RNA-Sequenz nach Anspruch 2 oder 3 codiert wird, oder das durch die Wirtszelle nach einem der Ansprüche 6 bis 9 hergestellt wird, oder ein Salz dieses Bindeproteins.

11. IFN-α/β-Bindeprotein nach Anspruch 10, das ein reifes Protein oder ein Salz davon ist.

12. Arzneimittel, umfassend das IFN-α/β-Bindeprotein nach Anspruch 10 oder 11 oder ein Salz davon, gegebenenfalls in Kombination mit einem pharmazeutisch verträglichen Träger.

13. Verwendung des IFN-α/β-Bindeproteins nach Anspruch 10 oder 11 für die Herstellung eines Arzneimittels zur Modulierung der IFN-α- oder IFN-β-Aktivität.

14. Verwendung des IFN-α/β-Bindeproteins nach Anspruch 10 oder 11 für die Herstellung eines Arzneimittels zur Inhibierung der IFN-α- oder IFN-β-Aktivität.

15. Verfahren zur Herstellung des IFN-α/β-Bindeproteins nach einem der Ansprüche 10 oder 11, umfassend die Züchtung der Wirtszelle nach einem der Ansprüche 6 bis 9 und die Gewinnung des IFN-α/β-Bindeproteins.

16. Diagnostische Zusammensetzung, umfassend das DNA-Molekül nach Anspruch 1 und/oder das IFN-α/β-Bindeprotein nach Anspruch 10 oder 11.

## Revendications

1. Molécule d'ADN codant pour une protéine se liant à IFN-α/β, choisie parmi
(a) IFNAB-BPI ayant la séquence d'aminoacides représentée sur la figure 2 ;
(b) IFNAB-BPII ayant la séquence d'aminoacides représentée sur la figure 5 ; et
(c) des précurseurs ou des protéines de fusion des protéines de (a) ou (b).

2. Molécule d'ARNm qui est le produit de transcription de la molécule d'ADN de la revendication 1.

3. Molécule d'ARNm selon la revendication 2, choisie parmi
(a) une molécule d'ARNm d'environ 1,5 kb qui, une fois traduite, produit un précurseur de IFNAB-BPI et
(b) une molécule d'ARNm d'environ 4,5 kb qui, une fois traduite, produit un IFNAB-BPII.

4. Véhicule d'expression apte à la réplication, comprenant la molécule d'ADN selon la revendication 1.

5. Véhicule d'expression apte à la réplication selon la revendication 4, qui est le plasmide pEF-BOS-IFNAB-BPI, dont la construction est décrite dans l'exemple 7.

6. Cellule hôte transformée par le vecteur d'expression apte à la réplication selon la revendication 4 ou 5.

7. Cellule hôte selon la revendication 6, qui est une cellule procaryote.

8. Cellule hôte selon la revendication 6, qui est une cellule eucaryote.

9. Cellule hôte selon la revendication 8, qui est une cellule COS simienne.

10. Protéine se liant à IFN-α/β, codée par la séquence d'ADN de la revendication 1 ou la séquence d'ARN de la revendication 2 ou 3 ou qui est produite par la cellule hôte de l'une quelconque des revendications 6 à 9, ou un sel de ladite protéine de liaison.

11. Protéine se liant à IFN-α/β selon la revendication 10, qui est une protéine mature ou un sel de celle-ci.

12. Composition pharmaceutique comprenant la protéine se liant à IFN-α/β selon la revendication 10 ou 11, ou un sel de celle-ci, éventuellement en association avec un véhicule pharmaceutiquement acceptable.

13. Utilisation de la protéine se liant à IFN-α/β selon la revendication 10 ou 11, pour la préparation d'une composition pharmaceutique destinée à la modulation de l'activité d'IFN-α ou d'IFN-β.

14. Utilisation de la protéine se liant à IFN-α/β selon la revendication 10 ou 11, pour la préparation d'une composition pharmaceutique destinée à l'inhibition de l'activité d'IFN-α ou d'IFN-β.

15. Procédé pour la production de la protéine se liant à IFN-α/β selon la revendication 10 ou 11, comprenant la culture de la cellule hôte selon l'une quelconque des revendications 6 à 9 et la récupération de ladite protéine se liant à IFN-α/β.

16. Composition de diagnostic, comprenant la molécule d'ADN de la revendication 1 et/ou la protéine se liant à IFN-α/β selon la revendication 10 ou 11.
